# EUROPÄISCHE PATENTSCHRIFT

(11) **EP 2 679 275 B1**
(45) Veröffentlichungstag und Bekanntmachung des Hinweises auf die Patenterteilung: **14.05.2014**
(21) Anmeldenummer: 12004848.3
(22) Anmeldetag: 28.06.2012
(51) Int. Cl.: A61N 1/05, A61B 5/042

(54) **Epikardiale Mapping-Elektrode**
Epicardial mapping electrode
Électrode épicardique de cartographie

(43) Veröffentlichungstag der Anmeldung: 01.01.2014
(73) Patentinhaber: Peter Osypka Stiftung, 79639 Grenzach-Wyhlen (DE)
(72) Erfinder: Osypka, Peter, 79639 Grenzach-Wyhlen (DE)

(56) Entgegenhaltungen:
- EP-A1- 2 241 279
- US-A- 5 607 462
- US-A- 5 849 033
- US-A1- 2002 002 329
- US-B1- 7 856 260

## Beschreibung

### Erfindung:

Die Erfindung betrifft eine Epikardiale Mapping Elektrode, die nach einer Herzoperation den linken Ventrikel an unterschiedlichen Stellen temporär überwacht und stimuliert, um eine optimale Position einer Elektrodenlage für eine permanente biventrikuläre Stimulation (Resynchronisation) zu selektieren.

### Probleme:

Die biverrtrikuläre Stimulation, auch unter dem medizinischen Begriff Resynchronisation bekannt, ist seit einigen Jahren eine Methode um bei Patienten mit einem geschwächten Herzen deren Herzleistung zu verbessern. Zu diesem Zweck wird ein Herzschrittmacher implantiert, der nicht nur wie bisher den rechten Ventrikel, sondern zusätzlich auch den linken Ventrikel des Herzens stimulieren kann. Der Erfolg einer solchen Therapie hängt u.a. von der richtigen Position der Elektrode auf der linken Seite des Herzens ab. Da die Implantation der Elektrode nicht direkt in den linken Ventrikel positioniert werden kann, benutzt man als Ersatz eine Position in einem Zweig des Koronar Sinus. Neben der sicheren Fixierung spielt die Position der Elektrode in den einzelnen Abschnitten des Koronar Sinus eine entscheidende Rolle für den Erfolg der Stimulations Therapie.

Das Patent US-A-5 607 462 zeigt eine Elektrode, die geeignet ist nach einer Herzoperation am geschlossenen Thorax den linken Ventrikel an unterschiedlichen Stellen temporär abzutasten und zu stimulieren, und zum epikardialen Mapping geeignet ist. Die Elektrode besteht aus einem flexiblen Material in Form eines Schlauches mit mehreren Lumen und ist pro Lumen mit einem Elektrodenpol ausgestattet, wobei der Schlauch auf der dem Herzen zugewandten Seite mindestens eine Vielzahl von Öffnungen besitzt, die als Fenster für den Elektroden-Pol dienen, derart das der Elektrodenpol in der jeweiligen Öffnung frei liegt.

### Lösungsweg:

Es besteht daher die Aufgabe, eine Elektrodenanordnung nach der eingangs beschriebenen Art zu schaffen, mit deren Hilfe nach einer Herzoperation selektiv, an unterschiedlichen Stellen des Herzens durch die temporäre Stimulation des linken Ventrikels über einen Zeitraum von einigen Tagen, geprüft werden kann, in wieweit die Verbesserung der Herzleistung erreicht werden kann. Nach zufriedenstellender Selektion des Areals für eine optimale permanente biventrikuläre Stimulation, kann die Stelle markiert und/oder prophylaktisch eine permanente Herzschrittmacher Elektrode implantiert werden. Die vorliegende Erfindung betrifft eine epikardiale Mapping Elektrode gemäß Anspruch 1 und eine Elektrodenanordnung gemäß Anspruch 8. Die Elektrodenanordnung selbst kann leicht durch eine Öffnung in der Brustwand, wie bei den Herzdrähten, nach Gebrauch herausgezogen werden.

### Vorteil:

Der Vorteil der Erfindung liegt darin, dass man auf einfache Weise nach einer Herzoperation am geschlossenen Thorax, den linken Ventrikel epikardial durch elektrische Stimulation intensiv Überwachen kann und insbesondere Areale setektieren kann, die später für biventrikuläre Stimulation optimal geeignet sind.

### Abbildungen:

Weitere Einzelheiten, Merkmale und Vorteile der Erfindung lassen sich dem folgenden Beschreibungsteil entnehmen, in dem anhand der Zeichnungen und Ausführungsbeispiele die Erfindung näher erläutert wird. Sie zeigen in schematischer Darstellung in
Fig.1. Eine beispielhafte Ansicht einer epikardialen Elektrodenanordnung auf dem Herzen mit den dazu gehörigen Polen.
Fig.2. Eine beispielhafte Ansicht einer Elektrodenanordnung mit zwei, zwischen einer Wendel parallel angeordneten Schtauchabschnitten mit Öffnungen und Zig-Zag und V-förmigen Fäden, die eine Fixierung der Elektrode auf der Herzoberfläche ermöglichen.
Fig.3. Eine weitere beispielhafte Ansicht einer Elektrodenanordnung mit einem, zwischen zwei Wendeln parallel angeordneten Schlauchabschnitt mit Öffnungen und V-förmigen Fäden, die eine Fixierung auf der Herzoberfläche ermöglichen.
Fig.4. Eine beispielhafte Ansicht einer erfindungsgemäßen Elektmdenanordnung, die nach den Merkmalen der Anatomie des Herzens in verschiedenen Ausführungen geformt werden kann.

### Beschreibung der Abbildungen:

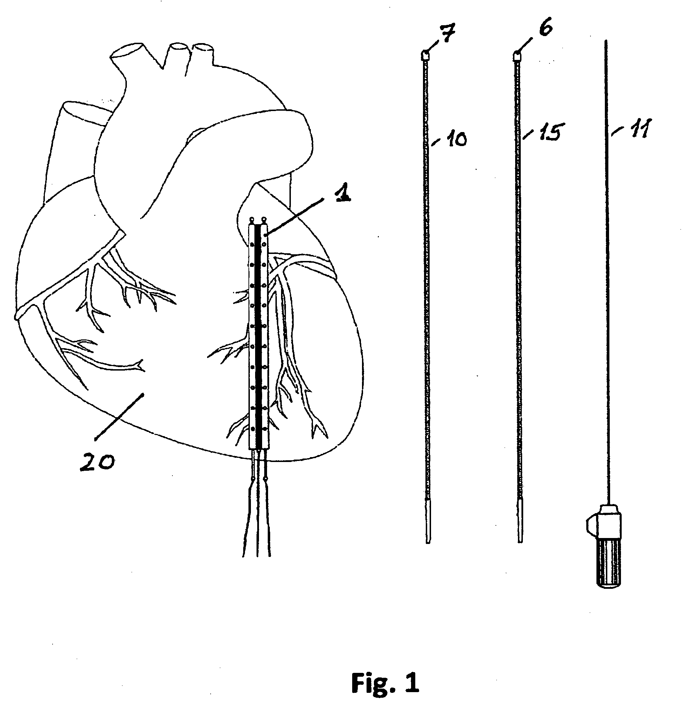

Fig.1. Eine beispielhafte Ansicht einer erfindungsgemäßen epikardialen Eiektrodenanordnung(1) auf dem Herzen(20) mit den dazu gehörigen Polen (6)(7), die distal auf einer isolierten Wendel (10)(15) integriert sind. Ein Stilett (11) sorgt in der Wendel für eine gewünschte Versteifung beim Manipulieren in der Elektroden Anordnung. Der Operateur entscheidet nach der Operation bei geschlossenem Herzen und offenem Thorax an welcher Stelle die Elektroden Anordnung platziert wird.

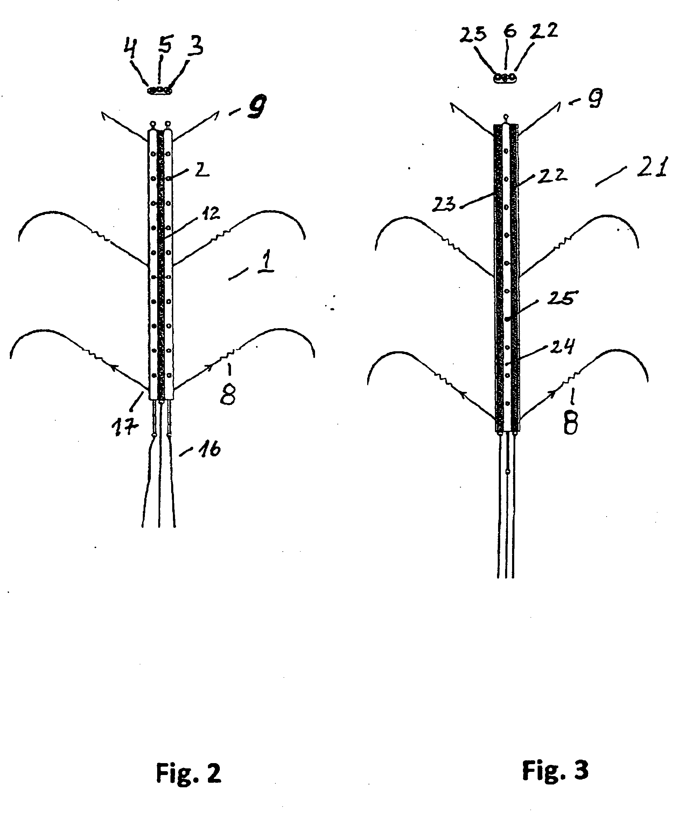

Fig.2. Zeigt eine beispielhafte Ansicht einer erfindungsgemäßen epikardialen Elektrodenanordnung (1) in einer dem Herzen zugewandten Seite in Schlauchform mit zwei Lumen (3)(4) und Öffnungen (2) für mindestens zwei gegeneinander verschiebbare Elektroden Pole (6)(7) zum Abtasten der Oberfläche des Herzens.

Die wendelförmigen monopolaren Elektroden (15 und 10) in den beiden Lumen der Elektroden Anordnung(3)(4) bestehen jeweils aus einem biegsamen isolierten Schaft z.B. einer Wendel und einem distalen Elektrodenkopf (6). Werden die gegeneinander verschiebbaren Elektroden Pole (6 und 7) in die schlauchförmige Elektrodenanordnung eingeführt, so werden immer nur die Potentiale der Herzoberfläche gemessen, an denen die Elektroden Pole (6 und 7) in der jeweiligen Öffnung (2) frei liegen. Durch Verschieben der Elektroden Pole kann so ein Areal auf der Oberfläche des Herzens abgetastet werden.

Die Vertiefung in der Schlauchform (5) dient zur Aufnahme einer Wendel(12), die als indifferenter Pol benutzt wird. Die Lumen 3 und 4 enthalten isolierte Wendeln (10)(15) an deren distalen Teil die Pole integriert sind.

Die zu messenden Potentiale werden über Leitungen (16) durch eine Öffnung im Brustkorb des Patienten nach außen geführt. Durch temporäre parallele Implantation mehrerer derartiger Elektrodenanordnungen können große Areale des Herzens vermessen werden. Bei einer gewünschten monopolaren Anordnung kann eine weitere indifferente großflächige Elektrode zB. in Form einer blanken Wendel an einer beliebigen Stelle im Brustraum des Patienten gelegt werden.

Ein Stilett (11) in jeder Wendel dient zur Versteifung der Wendel (15) und damit besseren Steuerung bei der Positionierung der Pole unter den Öffnungen zur Aufnahme von elektrischen Potentialen vom Herzen.

Außen am Schlauch der Elektrode können Fixierungselemente angebracht werden, die zur Fixierung der Elektrode auf der Herzoberfläche dienen. Derartige Fixierungselemente sind in Fig. 2,3 und 4 zu sehen.
In Fig. 2 sind an der Seite des Schlauchabschnittes (17) isolierte Litzen mit Abschnitten (8) in Zig-Zag Form angebracht oder mit Abschnitten (9) in V-Form. Dies sind Beispiele für Fixierungselemente. Die Fixierung ist jedoch nicht auf diese Beispiele beschränkt.

Die Elektrodenanordnung besteht aus einem flexiblen Material. Geeignete Materialien sind für alle Ausführungsformen beispielsweise Silikon, Dacron, Polyurethan, Polyester, Seide und dergleichen. Auch weitere biokompatible Kunststoffe wie z.B. Polyimid sind geeignet.

Die Elektrodenanordnung kann unterschiedliche Strukturen aufweisen. So kann in dem Schlauchteil beispielsweise ein Geflecht, ein Gewebe oder eine Folie integriert sein.

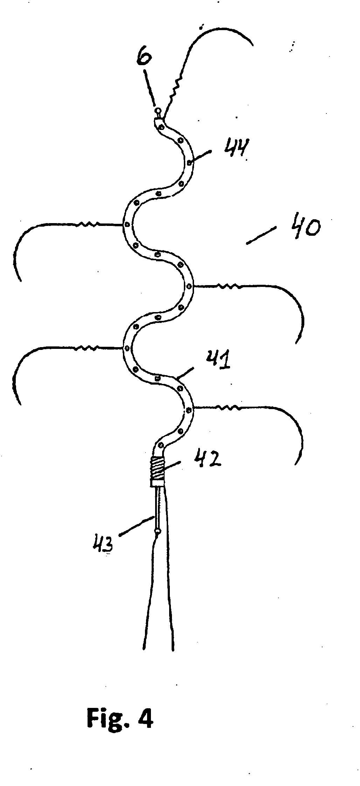

Die Elektrodenanordnung lässt sich an die Anatomie des Herzens anpassen(Fig.4). Die Formgebung wird durch einen aus einem Material mit Formgedächtnis bestehenden Draht, vorzugsweise Nitinol, unterstützt.

Die Elektrodenanordnung hat in der dem Herzen zugewandte Seite mehrere Öffnungen, die je nach Pol Größe und Pol-Form unterschiedlich sein können.

Die Elektrodenanordnung hat mindestens ein Lumen. In einer bevorzugten Ausfühningsförm hat die Elektrodenanordnung mehrere Lumen.
Die Position der Pole und deren Öffnungen sind variabel, sodass eine Anordnung entsprechend der anatomischen Lage der Vorhöfe möglich ist.

Geeignete Materialien für die Pole sind beispielsweise Edelstahl, MP35 N, Gold, Platin, Karbon, Nitinol und dergleichen. Auch ein leitfähiger mit Nanopartikeln gefüllter biokompatibler Kunststoff ist geeignet.

Fig.3.Zeigt eine weitere beispielhafte Ansicht einer erfindungsgemäßen mehrpoligen Elektrodenanordnung (21) mit einem, zwischen zwei Wendeln(22)(23) parallel angeordneten Schlauchabschnitt(24) mit Öffnungen(25).

Fig.4. Zeigt eine weitere beispielhafte Ansicht einer erfindungsgemäßen Elektrodenanordnung (40), die entsprechend der Anatomie des Herzens in verschiedenen Ausführungen geformt (41) werden kann. Die indifferente Elektrode ist am proximalen Ende des Schaftes in Form einer Wendel (42) angebracht. Der differente Elektroden Pol (6) wird durch Zug am proximalen Ende (43) der Elektrodenanordnung unter die entsprechenden Öffnungen (44) gezogen und ist an dieser Stelle als Elektrode wirksam.

Wie oben ausgeführt, unterliegt die Ausgestaltung der Pole einer großen Varianz (mehrere Materialien, eine Vielzahl von Formen). Es ist daher vorstellbar, dass die Gestaltung der Pole auf der Elektrodenanordnung als Kombination der oben genannten Eigenschaften in unterschiedlicher Form ausgeführt werden kann.

## Patentansprüche

1. Fpikardiale Mapping Elektrode, die geignet ist nach einer Herzoperation am geschlossenen Thorax den linken Ventrikel an unterschiedlichen Stellen temporär abzutasten und zu stimulieren wobei die Elektrode aus einem flexiblen Material in Form eines Schlauches besteht und der Schlauch mit mindestens einem Lumen, vorzugsweise mit mehreren Lumen und pro Lumen mit einem Elektrodenpol ausgestattet ist und wobei der Schlauch auf der dem Herzen zugewandten Seite eine Vielzahl von Öffnungen besitzt, die entlang des Schlauches derart angeordnet sind, dass die Öffnungen auf einer Linie liegen, die koaxial mit der Schlauchlängsachse ist, wobei die Öffnungen als Feneterfur den Elektrodenpol dienen, derart dass der Elektrodenpol in der jeweiligen Öffnung (2) frei liegt.

2. Epikardiale Mapping-Elektrode nach Anspruch 1, **dadurch gekennzeichnet, dass** das flexible Material aus Silikon, Dacron, Polyurethan, Polyester, Seide oder Polyimid besteht

3. Epikardiale Mapping-Elektrode nach Anspruch 1 - 2, **dadurch gekennzeichnet, dass** zwei gegeneinander verschiebbare Elektrodenpole vorhanden sind.

4. Epikardiale Mapping-Elektrode nach Anspruch 1 - 3, **dadurch gekennzeichnet, dass** sich in dem Lumen mindestens eine isolierte Wendel befindet an deren distalen Ende der Elektrodenpol fixiert ist.

5. Epikardiale Mapping-Elektrode nach Anspruch 1 bis 4, **dadurch gekennzeichnet, dass** sich innerhalb der Wendel ein Stilett befindet

6. Epikardiale Mapping-Etektrode nach Anspruch 1 bis 5, **dadurch gekennzeichnet, dass** Außen am Schlauch Fixierungselemente angebracht sind.

7. Epikardiale Mapping-Elektrode nach Anspruch 1 bis 6, **dadurch gekennzeichnet, dass** die Elektrode entsprechend der Anatomie des Herzens geformt ist und durch einen aus einem Material mit Formgedächtnis bestehenden Draht, vorzugsweise Nitinoldraht in der Formgebung unterstützt wird.

8. Elektrodenanordnung bestehend aus einer epikardialen Mapping-Elektrode nach Anspruch 1 bis 7, und mindestens einer indifferenten Elektrode.

9. Elektrodenanordnung nach Anspruch 8, wobei die indifferente Elektrode die Form einer Wendel besitzt, die sich über den gesamten Schlauch erstreckt oder am proximalen Ende des Lumens sich befindet und/oder als separate Wendel im Brustraum des Patienten untergebracht ist.

10. Elektrodenanordnung nach Anspruch 8 mit zwei indifferenten Elektroden in Form einer Wendel, wobei sich die epikardiale Mapping-Elektrode zwischen den indifferenten Elektroden befindet.

## Claims

1. An epicardial mapping electrode suitable for temporarily mapping and stimulating the left ventricle at different parts after a cardiac surgery with the thorax closed, whereby the electrode consists of a tube made of flexible material wherein the tube has at least one lumen, preferably more than one lumen, an electrode pole is present inside each lumen and wherein the tube has a number of openings facing the surface of the heart, said openings being arranged along the tube such that the openings are in direct line which is coaxial to the longitudinal axis of the tube , whereby the openings are intended to provide a window for the electrode pole so that the electrode pole is entirely exposed in the respective opening (2).

2. The epicardial mapping electrode according to claim 1 wherein the flexible material is selected from the group consisting of silicone, Dacron, polyurethane, polyester, silk and polyimide.

3. The epicardial mapping electrode according to claim 1-2 wherein two mutually displaceable electrode-pols are present.

4. The epicardial mapping electrode according to claim 1-3 wherein at least one isolated coil having at its distal end an electrode pole is positioned inside the lumen.

5. The epicardial mapping electrode according to claim 1-4 wherein a stylet is placed inside the coil electrode.

6. The epicardial mapping electrode according to claim 1-5 wherein fixing elements are placed at the outside of the flexible tube.

7. The epicardial mapping electrode according to claim 1-6 wherein the electrode is shaped according to the anatomy of the heart and wherein the design is supported by a shape memory metal wire, preferably by a nitinol wire.

8. An electrode assembly consisting of an epicardial mapping electrode according to claim 1-7 and at least one indifferent electrode.

9. The electrode assembly according to claim 8 wherein the indifferent electrode is in form of a coil either extending longitudinally through the entire tube or being positioned at the proximal end of the lumen and/or the indifferent electrode is a separate coll being placed In the thorax area of the patient.

10. The electrode according to claim 8 wherein two indifferent electrodes In form of a coil are present and whereby the epicardial mapping electrode is located between the indifferent electrodes.

## Revendications

1. Électrode épicardique de cartographie, destinée à être utilisée après une opération au coeur à thorax fermé convenant à cartographier et à stimuler temporairement le ventricule gauche à des endroits différents. L'électrode consiste en un matériau flexible en forme de tuyau, lequel est équipé d'au moins une lumière, de préférence de plus d'une lumière, et avec un pôle d'électrode par lumière, où le tuyau du côté situé face au coeur dispose d'un grand nombre d'ouvertures agencées le long du tuyau de manière à ce que les ouvertures forment une ligne coaxiale avec l'axe longitudinal du tuyau, dont les ouvertures servent de fenêtres au pôle d'électrode de manière à ce que le pôle d'électrode soit entièrement exposé dans l'ouverture (2) correspondante.

2. Électrode épicardique de cartographie conformément à la revendication 1, **caractérisée par le fait que** le matériau flexible soit en silicone, en Dacron, en polyurétane, en polyester, en sole ou en polylmide.

3. Électrode épicardique de cartographie conformément à la revendication 1 - 2, **caractérisée par le fait que** deux pâles d'électrodes mutuellement déplaçables sont présents.

4. Électrode épicardique de cartographie conformément à la revendication 1- 3, **caractérisée par le fait qu'**au moins une tige hélicoïdale isolée se trouve dans la lumière à l'extrémité distale de laquelle le pôle d'électrode est fixé.

5. Électrode épicardique de cartographie conformément à la revendication 1 - 4, **caractérisée par le fait qu'**un stylet se trouve à l'intérieur de la tige hélicoïdale.

6. Électrode épicardique de cartographie conformément à la revendication 1-5, **caractérisée par le fait que** des éléments de fixation sont montés à l'extérieur du tuyau.

7. Électrode épicardique de cartographie conformément à la revendication 1 - 6, **caractérisée par le fait que** l'électrode est formée conformément à l'anatomie du coeur et dont la forme est soutenue par un câble métallique constitué d'un matériau à mémoire, de préférence un câble en nitinol.

8. Dispositif d'électrodes consistant en une électrode épicardique de cartographie conformément à la revendication 1-7 et d'un moins une électrode indifférente.

9. Dispositif d'électrodes conformément à la revendication 8, où l'électrode Indifférente possède la forme d'une tige hélicoïdale s'étendant sur toute la longueur du tuyau ou se trouvant à l'extrémité proximale de la lumière et/ou est placée en tant que tige hélicoïdale séparée dans la cage thoracique du patient.

10. Dispositif d'électrodes conformément à la revendication 8 avec deux électrodes indifférentes en forme d'une tige hélicoïdale où l'électrode épicardlque de cartographie se trouve entre les électrodes Indifférentes.
